Europäisches Patentamt

**European Patent Office**

Office européen des brevets

(11) Veröffentlichungsnummer: **0 007 597**
**B1**

(12) EUROPÄISCHE PATENTSCHRIFT

(45) Veröffentlichungstag der Patentschrift:
16.01.85

(21) Anmeldenummer: 79102577.8

(22) Anmeldetag: 20.07.79

(51) Int. Cl.⁴: **A 61 K 31/195**, A 61 K 45/06,
A 61 K 31/415 // (A61K45/06,
31/195),(A61K45/06, 31/415)

(54) N-Niedrigalkylglycinamide und Sarkosinanhydrid sowie in Kombination mit bekannten tumorhemmenden Verbindungen vorliegendes Sarkosin zur Anwendung bei der Heilung von Tumoren sowie dieselben enthaltendes Mittel und Verfahren zu dessen Herstellung.

(30) Priorität: 20.07.78 DE 2832009

(43) Veröffentlichungstag der Anmeldung:
06.02.80 Patentblatt 80/3

(45) Bekanntmachung des Hinweises auf die Patenterteilung:
16.01.85 Patentblatt 85/3

(84) Benannte Vertragsstaaten:
AT BE CH FR GB IT LU NL SE

(56) Entgegenhaltungen:
BE - A - 647 556
DE - C - 553 424
FR - A - 2 085 637

CHEMICAL ABSTRACTS, Band 62, No. 3, veröffentl. am
1. Februar 1965, 3296fg, Columbus, Ohio, USA, v.A.
CHERNOV et al. "Antitumor acitivty of some amines and
amino acids of the indole series"
CHEMICAL ABSTRACTS, Band 74, No. 5, veröffentl. am
1. Februar 1971, 20129e, Seite 81, Columbus, Ohio, USA,
V.G. OVAKIMOV et al., "Protective action of biogenic
amines in a radiation intestinal syndrome"
CHEMICAL ABSTRACTS, Band 82, No. 25, veröffentl. am
23. Juni 1975, 164694y, Seite 12, Columbus, Ohio, USA,
c.A. APFFEL et al., "Tumor rejection in experimental
animals treated with radioprotective thiols"

(73) Patentinhaber: **Stiftung Deutsches
Krebsforschungszentrum, Im Neuenheimer Feld 280,
D-6900 Heidelberg 1 (DE)**

(72) Erfinder: **Osswald, Hans, Prof. Dr. med., Dürerstrasse 20,
D-6900 Heidelberg (DE)**
Erfinder: **Youssef, Mahmoud, Kolbenzeil 22,
D-6900 Heidelberg (DE)**

(74) Vertreter: **Deufel, Paul, Dr. et al, Patentanwälte
Müller-Boré, Deufel, Schön, Hertel Lewald, Otto
Isartorplatz 6 Postfach 26 02 47, D-8000 München 26 (DE)**

(56) Entgegenhaltungen: (Fortsetzung)
Hackh's Chemical Dictionary, 4. Ed. page 598

Die Akte enthält technische Angaben, die nach dem
Eingang der Anmeldung eingereicht wurden und die
nicht in dieser Patentschrift enthalten sind.

## Beschreibung

Es besteht ein starkes Bedürfnis für die Verbesserung der Chemotherapie von bösartigen Tumoren. Bisher erfolgte die Behandlung von Tumoren im wesentlichen durch antineoplastische Chemotherapeutika, wobei insbesondere die Antimetabolite, die Alkylantien und intercalierenden Stoffe sowie Pflanzeninhaltsstoffe zu nennen sind. Da diese Präparate nicht nur Tumorzellen, sondern auch andere rasch profilierende Systeme des Organismus, z.B. Blutbildungszentren, Haut- und Anhangsgebilde, Schleimhaut und Keimepithel beeinflussen, wird der therapeutische Erfolg durch unerwünschte Wirkungen begrenzt.

Die Steigerung der chemotherapeutischen Wirkund ohne Steigerung des toxischen Effektes ist demnach ein erstrebenswertes Ziel.

Es wurde nun gefunden, dass N-Niedrigalkylglycine mit 1 bis 4 Kohlenstoffatomen im N-Alkylteil in Form der Säureamide und Sarkosinanhydrid einzeln oder im Gemisch sowie in Kombination mit bekannten tumorhemmenden Verbindungen, wobei vor allem die Alkylantien und intercalierenden Stoffe zu nennen sind, bei Anwendung zur Heilung verschiedener Tumoren eine günstige Wirkung zeigen. Das gleiche gilt für Sarkosin in Kombination mit bekannten tumorhemmenden Verbindungen.

Sarkosin in Kombination mit fünf weiteren Aminosäuren ist ein aus FR-A 2 085 637 bekanntes Arzneimittel zur Behandlung von Asthenie. In Hackh's Chemical Dictionary, 4. Ed., Seite 598 wird angegeben, dass Sarkosin als Antirheumatikum verwendbar ist.

In den erfindungsgemässen Mitteln liegen die N-Niedrigalkylglycinamide und Sarkosinanhydrid einzeln, im Gemisch bzw. zusammen mit bekannten tumorhemmenden Verbindungen, oder auch Sarkosin zusammen mit bekannten tumorhemmenden Verbindungen, als aktive Komponente, ggf. mit üblichen Träger- und Hilfsstoffen, vor.

Die erfindungsgemässe Herstellung dieser Antitumormittel erfolgt durch Kombination von a) wenigstens einem Mitglied der Gruppe N-Niedrigalkylglycine mit 1 bis 4 Kohlenstoffatomen im N-Alkylteil in Form der Säureamide, Sarkosin und Sarkosinanhydrid mit b) einer bekannten Antitumorverbindung, insbesondere der Art, die als Cytostatika bekannt ist. Solche Antitumorverbindungen sind z.B. in Ullmanns' Enzyklopädie der technischen Chemie, 4. Auflage, Band 9, Seiten 705 bis 714, unter der Bezeichnung «Cytostatika» beschrieben. Von besonderem Interesse in diesem Zusammenhang sind antineoplastische Alkylantien, intercalierende Mittel und Antimetaboliten.

Das Verfahren besteht darin, a und b zu kombinieren, indem man beide Komponenten in innigen Kontakt miteinander bringt, vorzugsweise in Lösung, im allgemeinen in Mengen von 2:1 bis 50:1 Gewichtsteilen, insbesondere 2:1 bis 10:1 Gewichtsteilen an a:b. Die erhaltene Kombination wird in an sich bekannter Weise isoliert, beispielsweise durch Vakuumtrocknung, Gefriertrocknung, oder jede andere geeignete Massnahme und kann in üblicher Weise zu Einheiten konfektioniert werden, die eine wirksame Menge enthalten, beispielsweise durch Einkapselung oder Granulierung, gewünschtenfalls zusammen mit üblichen Füllstoffen, Exzipientien und/oder anderen geeigneten Hilfsstoffen. Das Kombinationsprodukt kann auch zu Injektionspräparaten konfektioniert werden.

Es ist auch häufig möglich, nur eine der beiden Komponenten als Lösung in einem geeigneten Lösungsmittel zu verwenden und die andere Komponente in Pulverform in diese Lösung einzubringen und nach kräftigem Verrühren und kurzer Umsetzungszeit das erhaltene Produkt zu isolieren. Zweckmässig liegen jedoch beide Komponenten in Lösung vor, insbesondere im gleichen Lösungsmittel. Falls Lösungsmittel verwendet werden, die für Injektionspräparate nicht geeignet sind, müssen sie in bekannter Weise entfernt werden und man konfektioniert erst dann zu Injektionspräparaten. Die Löslichkeiten der Reaktionsteilnehmer sind bekannt und können einschlägigen Veröffentlichungen entnommen werden; jedoch können auch ohne weiteres durch wenige Versuche geeignete Lösungsmittel und Reaktionsbedingungen ermittelt werden. Wichtig ist es, die Vorschriften bezüglich der Stabilität der bekannten Cytostatika zu beachten, damit bei der Umsetzung oder Isolierung keine Zersetzung dieser Reaktionskomponente eintritt.

Wegen der hohen Toxizität der meisten Cytostatika ist es meistens zweckmässiger, die Präparate nach ihrer Toxizität $LD_{50}$ zu konfektionieren, indem man a und b derart kombiniert, dass im allgemeinen $1/10$ bis $1/20$ der $LD_{50}$ von b mit $1/20$ bis $1/100$ der $LD_{50}$ von a ($LD_{50}$ in mg pro kg) zur Herstellung einer Einheitsdosierung vorliegt. Die bevorzugten Einheitsdosierungen haben einen grossen Sicherheitsspielraum und enthalten somit etwa $1/15$ der $LD_{50}$ von b kombiniert mit $1/20$ bis $1/40$, insbesondere $1/25$ bis $1/30$ der $LD_{50}$ von a, wobei man für die Einheitsdosierung von den üblichen Durchschnittsgewichten der zu behandelnden Patienten ausgeht, die auf diesem Gebiet statistisch erfasst und bekannt sind.

Es kann manchmal bevorzugt sein, a und b derart einzukapseln oder in Form einer Kombinationstablette zu kombinieren, dass eine der Komponenten a oder b teilweise getrennt in einer Form vorliegt, die eine verzögerte Abgabe an den Körper gewährleistet, beispielsweise in einer getrennten Kapsel, die dann in der Gesamtkapsel enthalten ist, die auch das Kombinationspräparat enthält. Methoden dieser Art der Einkapselung oder Bereitung von Kombinationstabletten oder Dragees sind wohl bekannt. Bei dieser Arbeitsweise wird nur ein Teil der Reaktionspartner a und b vor der Einkapselung oder Tablettierung oder Granulierung umgesetzt, während ein Teil von a oder b getrennt vorliegt, so dass eine Differenzierung hinsichtlich der zeitlichen Abgabe an den Körper möglich ist.

Die Verwendung von Sarkosin und der genannten Sarkosinderivate in Kombination mit den genannten antineoplastischen Chemotherapeutika steigert deren therapeutische Wirkung tatsächlich überadditiv ohne Zunahme der toxischen Nebenwirkungen. Der angestrebte Erfolg ist oftmals am grössten, wenn die Wirkung der Komponenten in zeitlichem Abstand erfolgt, wobei gewöhnlich das antineoplastische Mittel vorher und Sarkosin oder das Sarkosinderivat später wirken gelassen werden, was bei der Anwendung des angegebenen Kombinationspräparats, dessen eine Komponente verzögert an den Körper abgegeben wird, ohne weiteres möglich ist. Insbesondere bei intercalierenden Mitteln hat sich jedoch die gleichzeitige Wirkung beider Komponenten als zweckmässig erwiesen.

Von Interesse ist auch die Tatsache, dass z.B. intracerebral (in das Gehirn) implantierte Tumoren, welche durch Cyclophosphamid nicht beeinflussbar sind, durch die Kombination von Cyclophosphamid und Sarkosin bei über 50% der Versuchstiere geheilt werden können. Es bietet sich somit die Möglichkeit zu einer erheblichen Verbesserung der therapeutischen Möglichkeiten in der Behandlung von Tumoren.

Diese Eigenschaft von Sarkosin und gewissen Sarkosinderivaten scheint recht spezifisch zu sein, da z.B. Sarkosin, Sarkosinanhydrid, N-Methylglycinamid und N-Propylglycinamid bei verschiedenen Transplantationstumoren eine tumorhemmende Wirkung zeigen, wogegen interessanterweise Kreatin, ein Isoharnstoffderivat des Sarkosins, unwirksam ist.

Die folgenden Beispiele erläutern die Erfindung. Die Tierversuche wurden an Mäusen bzw. an Ratten durchgeführt. In die Tabellen 2, 3, 5, 8 und 9 sind Ergebnisse von Versuchen aufgenommen, bei denen eine erfindungsgemäss verwendbare und eine weitere tumorhemmende Verbindung zeitlich versetzt appliziert wurden, um auf diese Weise Modellversuche für die Anwendung von erfindungsgemässen Kombinationspräparaten mit verzögerter Abgabe einer Komponente zu schaffen.

Beispiel 1

Wirkung von Sarkosinanhydrid und N-Alkylglycinamiden für sich allein und in Kombination mit Cyclophosphamid (das im folgenden mit CPA abgekürzt wird und unter dem Handelsnamen «Endoxan» bekannt ist) und von dessen Kombination mit Sarkosin (Ergebnisse in Tabellen 1 bis 3).

Zur Prüfung dieser Verbindungen bei der Leukämie P 388 dienten als positive Kontrollsubstanzen N-Propanolglycinamid und CPA (Serien 2 bzw. 8 und 9 der Tabelle 1).

Sarkosinanhydrid, hier und im folgenden als Sarkosin AH bezeichnet (Serie 3, 4) besass eine eindeutige chemotherapeutische Wirkung bei einmaliger Gabe (Serie 3), bei Anwendung von zwei Dosen im Abstand von 48 h nahm die Heilungs- und Überlebensrate zu. Hingegen erreichte N-Methylglycinamid schon bei einmaliger Dosierung ein Wirkungsoptimum (Serie 5), welches durch Gabe von zwei gleichen Dosen nicht mehr gesteigert wurde. N-Propylglycinamid (Serie 7) bewirkte ebenso wie N-Methylglycinamid bei Gabe einer Dosis einen deutlichen chemotherapeutischen Effekt. N-Propanolglycinamid erwies sich hinsichtlich der Heilungsrate (5 von 15 Tieren geheilt) und in der ILS (Lebensverlängerung der behandelten Serie im Vergleich zur Kontrollserie in Prozent) als weitaus geringer wirksam, obwohl es dem N-Propylglycinamid (Serie 7) nahe verwandt ist.

Die Dosierung für das einzelne Präparat kann variiert werden. Ausgedehnte Untersuchungen mit Sarkosin sowie Sarkosinanhydrid zeigen, dass in einem Dosierungsbereich von 150 bis 270 mg/kg das Wirkungsoptimum für Sarkosin lag, während Sarkosinanhydrid bei 200 bis 320 mg/kg die beste chemotherapeutische Wirkung ausübte. Erhöhungen über den angegebenen Bereich führten nicht mehr zu einer Steigerung der chemotherapeutischen Wirkung. Es ist auch die Anwendung einer äquitoxischen Dosierung von Sarkosin und seinen Derivaten in Betracht zu ziehen, wie die folgenden Ausführungen zeigen. Da bei den besprochenen Versuchen Cyclophosphamid als Vergleichssubstanz diente, erscheint es von Interesse, einen Vergleich zwischen der chemotherapeutisch verwendeten CPA-Dosierung 30 resp. 60 mg/kg und der Dosis letalis 50 (DL 50), welche für CPA bei subkutaner Anwendung 450 mg/kg beträgt, zu ziehen. Bei Zugrundelegung der geringsten therapeutisch verwendeten Dosis von CPA ergibt sich ein Faktor 15, d.h. das 15-fache der Dosierung von 30 mg/kg entspricht der DL 50 von CPA.

Für Sarkosin liegt die DL 50 bei 6500 mg/kg. Der Unterschied zwischen der kleinsten in einem solchen Versuch anwendbaren Dosis von Sarkosin (270 mg/kg) und der DL 50 von Sarkosin ergibt einen Faktor von 23, d.h. die 23-fach höhere Dosierung der therapeutisch verwendeten Sarkosindosis entspricht der DL 50 von Sarkosin. Für Sarkosinanhydrid ergab sich eine DL 50 von 8300 mg/kg. In dem verwendeten Testsystem (Leukämie P 388) bieten also Sarkosin und seine Derivate wegen der geringeren Toxizität Vorteile gegenüber CPA.

Wie schon erwähnt, besteht der Anwendungsbereich für Sarkosin und in bevorzugter Weise für die erwähnten Derivate in der Kombination mit anderen tumorhemmenden Mitteln, insbesondere mit Alkylantien und intercalierenden Substanzen, wobei von den Alkylantien vor allem Cyclophosphamid und 1,3-bis-(2-Chloräthyl)-1-nitrosoharnstoff sowie 1-(2-Chloräthyl)-3-(cyclohexyl)-1-nitrosoharnstoff und andere 2-Chloräthyl-1-nitrosoharnstoffderivate und als intercalierende Stoffe vor allem Adriamycin, Dactinomycin, Chlorambucil und Melphalan zu nennen sind. Für die Kombination mit Metaboliten ist vor allem Etoposid (Handelsname «Methotrexat») zu nennen.

Sarkosin und Sarkosinderivate mit freier Carboxylgruppe oder noch zur Salzbildung zur Verfügung stehendem basischem Stickstoff können als übliches Salz, z.B. Alkali- oder Ammoniumsalz, verwendet werden, was gewöhnlich die Löslichkeit verbessert.

Als Beispiel sei die Kombination von Sarkosin mit CPA bei Lewis-Lung-Carcinom der Maus erwähnt. Das Lewis-Lung-Carcinom stellt einen der wenigen transplantablen Tumoren dar, welche sich durch Metastasenbildung auszeichnen. Das Lewis-Lung-Carcinom ist ein Bronchialcarcinom der Maus, das nach Implantation in die Muskulatur des Hinterschenkels in relativ kurzer Zeit zu einer ausgedehnten Metastasenbildung in der Lunge führt. Daraus ergibt sich ein Tumormodell, welches hinsichtlich der Metastasenbildung dem biologischen Verhalten von Tumoren beim Menschen mehr entspricht.

Aus Tabelle 2 geht hervor, dass CPA in einmaliger steigender Dosierung (80, 120, 160 mg/kg) als auch in drei Dosen von 80 mg/kg pro Woche und 120 mg/kg pro Woche gegeben wurde (Serie 2 bis 6). Bei einmaliger Dosierung von 80 mg/kg CPA (Serie 2) betrug die Heilungsrate 2 von 15 Tieren, die Überlebensrate 110%.

Bei Kombination von 80 mg/kg CPA mit 270 mg/kg Sarkosin, wobei als Modellversuch die Kombinationspartner im Abstand von 6 Stunden verabfolgt wurden (Serie 7) sind 10 von 15 Tieren geheilt, die Überlebensrate belief sich auf 187%. Ein Vergleich der Wirkung der Heilungs- und Überlebensrate in der CPA-Sarkosin-Kombination (Serie 7) mit der chemotherapeutischen Wirkung der doppelten CPA-Dosis (Serie 5) zeigt, dass die Heilungsrate (4 von 15 Tieren) deutlich geringer ist. Die Berechnung des Unterschiedes der geheilten Tiere, welche 80 mg/kg CPA erhielten (Serie 2) im Vergleich zu CPA-Sarkosin-Kombination (Serie 7) im Vierfeldertest bei Vorgabe von $2\alpha \sim 0,5$ ergibt einen statistisch signifikanten Unterschied.

Die Bedeutung des Zeitintervalles zwischen der Wirkung von CPA und Sarkosin wird in Serie 8 und 9 sichtbar. Eine Verlängerung des Zeitintervalles auf 8 Stunden führt nur zu einer geringen Änderung der Heilungs- und Überlebensrate (Serie 8). Hingegen führt eine Verkürzung des Zeitintervalles auf 3 Stunden (Serie 9) zu einer deutlichen Abnahme der Heilungs- und Überlebensrate. Obwohl hinsichtlich der Verdoppelungszeit des Tumors als auch der Generationszeit der Tumorzellen erhebliche Unterschiede zwischen dem Lewis-Lung-Carcinom und dem Ehrlich-Carcinom resp. Sarkom 180 bestehen, erweist sich das Zeitintervall zwischen CPA resp. anderen Alkylantien mit 6 Stunden optimal, wobei ein allgemeiner Bereich von ca. 4 bis 18 Stunden genannt werden kann.

In Tabelle 3 sind wiederum Ergebnisse der Kombinationen mit Cyclophosphamid und Sarkosin dargestellt. Die Behandlung begann nach Erreichung eines durchschnittlichen Tumorgewichtes von 3,5 g, um härtere Testbedingungen zu erreichen. Als Tumormodell wurde das Ehrlich-Carcinom der Maus und eine Behandlungsdauer von vier Wochen gewählt. CPA erreicht bei einer Dosierung von 120 mg/kg (Serie 2) keine Tumorheilung, jedoch kommt es zu einer deutlichen Tumorhemmung. Bei Verdoppelung der CPA-Dosis auf 240 mg/kg (Serie 3) sind 5 von 15 Tieren geheilt. Sarkosin in Monotherapie (Serie 4) besitzt keine tumorhemmende Wirkung. Die Kombination von Sarkosin-CPA (Serie 5) führt zu einem signifikanten curativen Effekt (10 von 15 Tieren geheilt). Da CPA in allen Kombinationen in einer Dosierung von 120 mg/kg verwendet wurde, lässt sich erkennen, dass die Sarkosin-CPA-Kombination den curativen Effekt der verdoppelten CPA-Dosis in Serie 3 deutlich übertrifft. Die gleichzeitige Gabe von Sarkosin und CPA (Serie 6) zeigt eine Wirkungserhöhung gegenüber der CPA-Monotherapie (Serie 2).

Die CPA-Sarkosin-Kombination bei einem Zeitintervall von 3 Stunden (Serie 7) bringt einen Anstieg der curativen Wirkung. Einen signifikanten Unterschied gegenüber Serie 3 (CPA-Monotherapie) ergibt die CPA-Sarkosin-Kombination bei einem Zeitintervall von 6 Stunden (Serie 8, 12 von 15 Tieren geheilt). Bei längerem Zeitintervall (Serie 9, 18 Stunden) nimmt die curative Wirkung wieder ab. Auffällige Unterschiede zeigen sich ausserdem im Vergleich der Körpergewichtsdifferenz zwischen Versuchsbeginn und Versuchsende innerhalb der mit CPA behandelten Serie (siehe letzte Spalte) und den CPA-Sarkosin-Kombinationen. Es zeigt sich deutlich, dass der Verlust des Körpergewichtes bei den CPA-Sarkosin-Kombinationen geringer ist als bei den CPA-Serien.

Beispiel 2

Wirkung von Adriamycin als weitere tumorhemmende Verbindung für sich allein und in Kombination mit Sarkosin (Ergebnisse in Tabellen 4 und 5).

Tabelle 4 zeigt, dass Adriamycin in einer Dosis von 2,5 mg/kg intravenös (Serie 2) beim Sarkom 180 keine curative Wirkung und keine verwertbare Hemmung des durchschnittlichen Tumorgewichtes bewirkt; das durchschnittliche Tumorgewicht wurde um weniger als 50% gegenüber der Kontrolle (Serie 1) gehemmt. Die gleichzeitige Adriamycin-Sarkosin-Kombination (Serie 3) bewirkt, dass 6 von 10 Tieren geheilt wurden und bei den nichtgeheilten eine signifikante Hemmung des Tumorgewichtes bestand.

In Tabelle 5 wurde ebenfalls beim Sarkom 180 Adriamycin in erhöhter Dosierung (8 mg/kg iv.) angewendet (Serie 2). Bei dieser Dosierung kam es zu einer signifikanten Hemmung des Tumorwachstums gegenüber der Kontrollserie; der curative Effekt in dieser Serie (1 von 15 Tieren geheilt) war jedoch auch mit toxischen Nebenwirkungen begleitet (1 von 15 Tieren gestorben). Hingegen führt die Sarkosin-Adriamycin-Kombination mit Zeitintervall zu einer signifikanten Erhöhung der Heilungsrate (9 von 15 Tieren geheilt) ohne Todesrate durch toxische Wirkungen. Ein ähnlicher Trend zeigt sich beim Vergleich der Körpergewichtsdifferenz beider behandelter Serien. Es muss hierbei auffallen, dass bei allen Kontrollserien die Körpergewichtsdifferenz zwischen Versuchsbeginn und Versuchsende relativ hoch ist. Dies erklärt sich aus der Tumorkachexie, welche durch das fortschreitende, ungehemmte Tumorwachstum auftritt.

Wie weitere Untersuchungen zeigten, ergibt sich auch die Möglichkeit einer Kombination mit anderen tumorhemmenden antineoplastischen Chemotherapeutika, wobei der Vorteil auch dieser Kombinationen darin besteht, dass eine überadditive antineoplastische Wirkung ohne Zunahme der Toxizität erreicht wird, da Sarkosin und dessen Derivate nur eine geringe Toxizität aufweisen. Dies bietet die Möglichkeit für Kombinationspräparate zur gleichzeitigen Verabreichung von Sarkosin und/oder Sarkosinderivat, insbesondere mit intercalierenden Stoffen, wobei vor allem die Kombination Sarkosin-Adriamycin zu nennen ist.

In den folgenden Tabellen 1, 2 und 3 sind die in Beispiel 1 beschriebenen Versuche zusammengefasst und die Tabellen 4 und 5 geben die Ergebnisse der in Beispiel 2 beschriebenen Versuche wieder.

Tabelle 1

| Serie Nr. | Präparat | Einzel-dosis mg/kg | Gesamt-dosis mg/kg | Geheilt | Gestorben | ILS % | durchschnitt-liche Überlebenszeit |
|---|---|---|---|---|---|---|---|
| 1 | Kontrolle | – | – | – | 15/15 | – | 7 – 10,73 – 14 |
| 2 | N-Propanol-glycinamid sc. | 450 | 450 | 5/15 | 10/15 | 16,03 | 10 – 12,7 – 16 |
| 3 | Sarkosin AH sc. | 260 | 260 | 6/15 | 9/15 | 85,15 | 13 – 13,1 – 14 |
| 4 nach 48 h | Sarkosin AH sc. Sarkosin AH sc. | 260 260 | 260 260 | 8/15 | 7/15 | 106,89 | 13 – 13,28 – 15 |
| 5 | N-Methyl-glycinamid sc. | 318 | 318 | 9/15 | 6/15 | 112,48 | 10 – 12 – 13 |
| 6 nach 48 h | N-Methyl-glycinamid sc. N-Methyl-glycinamid | 318 318 | 318 318 | 8/15 | 7/15 | 100,02 | 10 – 11,71 – 13 |
| 7 | N-Propyl-glycinamid sc. | 380 | 380 | 9/15 | 6/15 | 113,73 | 10 – 12,3 – 15 |
| 8 | CPA sc. | 30 | 30 | 9/15 | 6/15 | 126,15 | 14 – 15,66 – 17 |
| 9 | CPA sc. | 60 | 60 | 10/15 | 5/15 | 107,51 | 14 – 18,4 – 22 |

| | | | |
|---|---|---|---|
| Stamm: | $D_2B_6F_1$ | Transpl.-Datum: | 16. 5. 1978 |
| Tumor: | P 388 | Therapiebeginn: | 17. 5. 1978 |
| Transpl.-Modus: | ip | Behandlungstage: | 1 × pro Woche |
| | | Therapiedauer: | 1 Woche |
| | | Versuchsende: | 9. 6. 1978 |
| | | Tierzahl/Serie: | 15 |

Tabelle 2

| Serie Nr. | Präparat | Einzel-dosis mg/kg | Gesamt-dosis mg/kg | Geheilt | Gestorben | ILS % | durchschnitt-liche Überlebenszeit |
|---|---|---|---|---|---|---|---|
| 1 | Kontrolle | – | – | – | 15/15 | – | 27 – 27 – 29 |
| 2 | CPA sc. 1 × Behandlung | 80 | 80 | 2/15 | 13/15 | 110,9 | 37 – 53,0 – 68 |
| 3 | CPA sc. 1 × Behandlung | 120 | 120 | 1/15 | 14/15 | 60,5 | 36 – 40,7 – 55 |
| 4 | CPA sc. | 120 | 360 | 3/15 | 12/15 | 139,3 | 47 – 59,6 – 61 |
| 5 | CPA sc. 1 × Behandlung | 160 | 160 | 4/15 | 11/15 | 135,9 | 47 – 55,6 – 70 |
| 6 | CPA sc. | 80 | 240 | – | 15/15 | 92,5 | 43 – 56,8 – 60 |
| 7 nach 6 h | CPA sc. Sarkosin sc. | 80 270 | 240 810 | 10/15 | 5/15 | 187,4 | 44 – 56,8 – 60 |
| 8 nach 8 h | CPA sc. Sarkosin sc. | 80 270 | 240 810 | 9/15 | 6/15 | 169,7 | 36 – 50,1 – 60 |
| 9 nach 3 h | CPA sc. Inosin sc. | 80 200 | 240 600 | 5/15 | 10/15 | 147,1 | 47 – 56,8 – 60 |

| Stamm: | $D_2B_6F_1$ | Transpl.-Datum: | 1. 3. 1978 |
|---|---|---|---|
| Tumor: | Lewis-Lung-Carcinoma | Therapiebeginn: | 6. 3. 1978 |
| | | Behandlungstage: | 1 × wöchentlich |
| Transpl.-Modus | i.m. | Therapiedauer: | 1 oder 3 Wochen |
| Tumorgew. bei | 1,2 g – Therapiebeginn nach 5 Tagen | Versuchsende: | 30. 4. 1978 |
| Behandlungsbeginn: | | Tierzahl/Serie: | 15 |

Tabelle 3

| Serie Nr. | Präparat | Einzel-dosis mg/kg | Gesamt-dosis mg/kg | Geheilt | Gestorben | $\varnothing$ Tumor-gewicht | $\varnothing$ Körpergewichts-differenz |
|---|---|---|---|---|---|---|---|
| 1 | Kontrolle | – | – | – | – | 16,9 | − 21,3% |
| 2 | CPA | 120 | 480 | – | – | 3,3 | − 15,3% |
| 3 | CPA | 240 | 960 | 5/15 | – | 2,4 | − 19,3% |
| 4 | Sarkosin | 280 | 1120 | – | – | 14,7 | − 21,1% |
| 5 | Sarkosin sc. | 280 | 1120 | 10/15 | – | 1,4 | − 7,7% |
| nach 4 h | CPA sc. | 120 | 480 | | | | |
| 6 | CPA + | 120 | 480 | 2/15 | – | 3,2 | − 7,1% |
| | Sarkosin | 280 | 1120 | | | | |
| 7 | CPA sc. | 120 | 480 | 4/15 | – | 2,4 | − 4,5% |
| nach 3 h | Sarkosin | 280 | 1120 | | | | |
| 8 | CPA sc. | 120 | 480 | 12/15 | – | 2,5 | − 2,8% |
| nach 6 h | Sarkosin | 280 | 1120 | | | | |
| 9 | CPA sc. | 120 | 480 | 6/15 | – | 3,1 | − 7,3% |
| nach 18 h | Sarkosin sc. | 280 | 1120 | | | | |

| Stamm: | Swiss | Transpl.-Datum: | 3. 3. 1977 |
|---|---|---|---|
| Tumor: | Ehrlich-dpl. | Therapiebeginn: | 8. 3. 1977 |
| Transpl.-Modus | i.m. | Behandlungstage: | 1 × wöchentlich |
| Tumorgew. bei | | Therapiedauer: | 4 Wochen |
| Behandlungsbeginn | 3,8 g | Versuchsende: | 4. 4. 1977 |
| | | Tierzahl/Serie: | 15 Mäuse |

Tabelle 4

| Serie Nr. | Präparat | Einzel-dosis mg/kg | Gesamt-dosis mg/kg | Geheilt | Gestorben | $\varnothing$ Tumor-gewicht | $\varnothing$ Körpergewichts-differenz |
|---|---|---|---|---|---|---|---|
| 1 | Kontrolle | – | – | – | – | 14,7 | − 26,3% |
| 2 | Adria-mycin i.v. | 2,5 | 10 | – | – | 8,9 | − 17,0% |
| 3 | Adriamycin | 2,5 | 10 | | | | |
| | + | | | 6/15 | – | 2,9 | − 10,1% |
| | Sarkosin i.v. | 200 | 800 | | | | |

| Stamm: | Swiss | Transpl.-Datum: | 21. 1. 1977 |
|---|---|---|---|
| Tumor: | Sarkom 180 | Therapiebeginn: | 24. 1. 1977 |
| Transpl.-Modus | i.m. | Behandlungstage: | 1 × wöchentlich |
| Tumorgewicht bei | | Therapiedauer: | 4 Wochen |
| Behandlungsbeginn | 1,2 g | Versuchsende: | 26. 2. 1977 |
| | | Tierzahl/Serie: | 15 Mäuse |

Tabelle 5

| Serie Nr. | Präparat | Einzel-dosis mg/kg | Gesamt-dosis mg/kg | Geheilt | Gestorben | ∅ Tumor-gewicht | ∅ Körpergewichts-differenz |
|---|---|---|---|---|---|---|---|
| 1 | Kontrolle | – | – | – | – | 15,1 | – 14,3% |
| 2 | Adramycin i.v. | 8 | 24 | 1/15 | 1/15 | 5,4 | – 9,9% |
| 3 | Sarkosin i.v. | 180 | 540 | 9/15 | – | 5,0 | – 3,5% |
| nach 2 h | Adriamycin i.v. | 8 | 24 | | | | |

| | | | |
|---|---|---|---|
| Stamm: | Swiss | Transpl.-Datum: | 29. 11. 1976 |
| Tumor: | Sarkom 180 | Therapiebeginn: | 1. 12. 1976 |
| Transpl.-Modus | i.m. | Behandlungstage: | 1 × wöchentlich |
| Tumorgewicht bei | | Therapiedauer: | 3 Wochen |
| Behandlungsbeginn | 0,7 ∅ | Versuchsende: | 28. 12. 1976 |
| | | Tierzahl/Serie: | 15 Mäuse |

Beispiel 3

Vergleich der chemotherapeutischen Wirkung von Sarkosinanhydrid (Sarkosin AH) sowie CPA beim intramuskulär implantierten DE-Carcinosarkom (Tab. 6).

Die Therapie ist bei weit fortgeschrittenem Tumorwachstum (Tumorgrösse 4,5 bis 5,0 Gramm) begonnen worden. Die Resistenz des Tumors gegenüber Cyclophosphamid lässt sich aus Serie 2 erkennen. Der Unterschied zwischen dem Tumorgewicht der Kontrollserie (Serie 1) und der CPAbehandelten Serie (28,6 g/20,1 g) ist sehr gering.

Sarkosin AH erreicht schon bei einmaliger, subcutaner Gabe von 290 mg/kg bei 10 von 10 Ratten einen curativen Effekt (Serie 3), das gleiche Resultat wird bei dreimaliger, subcutaner Gabe von 290 mg/kg Sarkosin AH erreicht (Serie 4). Anscheinend greift Sarkosinanhydrid in Vorstufen der Purinsynthese ein, wobei sich in Abhängigkeit von der Tumorart Unterschiede im Ausmass der Wirksamkeit ergeben. Die grosse Sensibilität des DS-Carcinosarkoms gegenüber Sarkosinanhydrid eröffnet die Möglichkeit, den biochemischen Wirkungsmechanismus zu klären und die Frage der fehlenden Toxizität auch bei höherer Dosierung gegenüber normalen, proliferierenden Systemen (z.B. Blutbildungszentren im Knochenmark) einer Lösung näherzubringen.

Die Ergebnisse sind in der folgenden Tabelle 6 zusammengefasst.

Tabelle 6

| Serie Nr. | Präparat | Einzel-dosis mg/kg | Gesamt-dosis mg/kg | Geheilt | Gestorben | ∅ ± Tumorgewicht |
|---|---|---|---|---|---|---|
| 1 | Kontrolle | – | – | – | – | 28,6 ± 1,7 |
| 2 | CPA i.v. | 60 | 120 | – | – | 20,1 ± 1,0 |
| 3 | Sarkosin AH sc. | 290 | 580 | 10/10 | – | – |
| 4 | Sarkosin AH sc. 3 × wöchent-lich Mo. Mit. Fr. | 290 | 1160 | 10/10 | – | – |

| | | | |
|---|---|---|---|
| Stamm: | SD-Ratten | Transpl.-Datum: | 12. 3. 1979 |
| Tumor: | DS-Carcinosarkom | Therapiebeginn: | 21. 3. 1979 |
| Transpl.-Modus: | intramuskulär | Therapiedauer: | 2 Wochen |
| Tumorgewicht bei | | Versuchsende: | 2. 4. 1979 |
| Behandlungsbeginn: | 4,5 bis 5,0 g | Tierzahl/Serie: | 10 |

Beispiel 4

Vergleich von Sarkosinanhydrid mit CPA beim DS-Carcinosarkom der Ratte (Tabelle 7).

Aus zahlreichen Angaben der Literatur (z.B. Brock, N.: «Experimental Basis of Cancer Chemo-therapy», Chemotherapy 7, 19 bis 50 [1976]) ist bekannt, dass das DS-Carcinosarkom durch CPA nur gering beeinflussbar ist. Im vorliegenden Versuch wurden CPA und Sarkosinanhydrid vergleichend geprüft. Einschränkend sei erwähnt, dass CPA in einer wöchentlichen Dosis von 80 mg/kg noch toleriert wird, während Sarkosinanhydrid bei täglicher, subcutaner Gabe von 2 × 200 mg/kg gut vertragen wird. Es zeigt sich, dass CPA in einer Dosierung von 60 mg/kg bei 2 von 10 Tieren eine Tumorheilung bewirkt, während die übrigen Tiere praktisch keine Hemmung des Tumorwachstums erfahren, wie es aus dem Vergleich der durchschnittlichen Tumorgewichte von Kontroll- und CPA-Serie (Serie 1, 2) hervorgeht. Sarkosinanhydrid führt bei einer Dosierung von 290 mg/kg sc. (3 × pro Woche) bei 8 von 10 Tieren zu einem völligen Tumorrückgang. Die Tumorhemmung bei den zwei nichtgeheilten Tieren erscheint unbedeutend.

Die Ergebnisse sind in der folgenden Tabelle 7 zusammengefasst.

Tabelle 7

| Serie Nr. | Präparat | Einzel-dosis mg/kg | Gesamt-dosis mg/kg | Geheilt | Gestorben | $\emptyset\pm$ Tumorgewicht |
|---|---|---|---|---|---|---|
| 1 | Kontrolle | – | – | – | – | 16,2 ± 1,48 |
| 2 | CPA sc. | | | | | |
| | 1 × pro Woche 60 | | 180 | 2/10 | – | 15,3 ± 2,1 |
| 3 | Sarkosin AH sc. | | | | | |
| | 3 × pro Woche 290 | | 2320 | 8/10 | – | 12,4 ± 1,2 |

| | | | |
|---|---|---|---|
| Stamm: | Sprague-Dawley | Transpl.-Datum: | 15. 2. 1979 |
| Tumor: | DS-Carcinosarkom | Therapiebeginn: | 21. 2. 1979 |
| Transpl.-Modus: | i.m. | Behandlungstage: | Mo, Mi, Fr |
| Tumorgewicht bei | | Therapiedauer: | 3 Wochen |
| Behandlungsbeginn: | $\emptyset$ 1,5 g | Versuchsende: | 9. 3. 1979 |
| | | Tierzahl/Serie: | 10 |

**Beispiel 5**

Vergleich der chemotherapeutischen Wirkung verschiedener Dosierungen von VM 26 und Sarkosinanhydrid sowie ihrer Kombination beim diploiden, intracerebral implantierten Ehrlich-Ascitestumor (Ergebnisse in Tabelle 8).

VM 26, ein Epipodophyllotoxinderivat, bei dem es sich um 4'-Demethylepipodophyllotoxin-thenyliden-glucosid handelt, wird bei akuter myeloischer Leukämie, bei neurogenen Tumoren und malignen Lymphomen eingesetzt, wobei insbesondere bei neurogenen Tumoren die bisherigen Ergebnisse interessant erscheinen.

Die Tests zeigten folgendes:

VM 26, dessen klinischer Anwendungsbereich Hirntumoren und Morbus Hodgkins umfasst, besitzt in Abhängigkeit von der Dosis beim intracerebral implantierten Ehrlich-Ascitestumor eine chemotherapeutische Wirkung (Serie 2 bis 4), welche allerdings erst bei einer Gabe 18 mg/kg/intravenös/Woche zu einer ausgeprägten curativen Wirkung (10 von 15 Mäusen geheilt) führt. Die Kombination von VM 26 (6 mg/kg) und Sarkosinanhydrid (290 mg/kg) übertrifft bei gleichzeitiger Gabe die curative Wirkung der dreifach höheren Monotherapie mit VM 26 (Serie 5). Alle Tiere sind geheilt. Hingegen ergibt sich eine abnehmende curative Wirksamkeit der VM 26-Sarkosinanhydrid-Kombination, wenn das Zeitintervall zwischen den Kombinationspartnern 3 oder 6 Stunden beträgt (Serie 6, 7). Sarkosinanhydrid besitzt bei einmaliger Gabe, wie es in der Kombination mit VM 26 verwendet wird, keinen Einfluss auf die Absterberate (Serie 8). Doch führt die an fünf aufeinander folgenden Tagen applizierte Dosis von Sarkosinanhydrid bei 13 von 15 Mäusen zur Heilung (Serie 9), während die Einzelgabe von 1150 mg/kg Sarkosinanhydrid bei 8 von 15 Mäusen zur Heilung führt (Serie 10).

Sarkosinanhydrid wird bei täglicher, subkutaner Gabe von 2 × 2500 mg/kg über 14 Tage ohne Gewichtsverlust toleriert, während VM 26 bei einer Dosierung von 18 mg/kg intravenös nur einmal pro Woche (insgesamt 14 Tage) ohne schwere toxische Schädigung gegeben werden kann. Es erscheint neben der Kombinationswirkung von Sarkosinanhydrid mit VM 26 von Interesse, dass bei alleiniger Verwendung einer höheren Dosierung oder täglicher Gabe von Sarkosinanhydrid ein deutlicher, curativer Effekt erreicht werden kann.

Tabelle 8

| Serie Nr. | Präparat | Einzel-dosis mg/kg | Gesamt-dosis mg/kg | Geheilt | Gestorben | durchschnittl. Überlebens-zeit |
|---|---|---|---|---|---|---|
| 1 | Kontrolle | – | – | 0/15 | 15/15 | 6 – 6, 5 – 8 |
| 2 | VM 26 i.v. | 6 | 12 | 3/15 | 12/15 | 6 – 9, 3 – 13 |
| 3 | VM 26 i.v. | 12 | 24 | 4/15 | 11/15 | 7 – 9, 7 – 13 |
| 4 | VM 26 i.v. | 18 | 36 | 10/15 | 5/15 | 7 – 11, 8 – 13 |
| 5 | VM 26 i.v. + | 6 | 12 | | | |
| | Sarkosin AH sc. | 290 | 580 | 15/15 | 0/15 | |
| 6 | VM 26 i.v. | 6 | 12 | | | |
| | 3 h später Sarkosin AH sc. | 290 | 580 | 13/15 | 2/15 | 6 – 7, 5 – 9 |
| 7 | VM 26 i.v. | 6 | 12 | | | |
| | 6 h später Sarkosin AH sc. | 290 | 580 | 9/15 | 6/15 | 6 – 10, 1 – 13 |
| 8 | Sarkosin AH sc. | 290 | 580 | 0/15 | 15/15 | 6 – 7, 6 – 9 |
| 9 | Sarkosin AH sc. | 5 × 290 | 2900 | 13/15 | 2/15 | 10 – 11, 5 – 13 |
| 10 | Sarkosin AH sc. | 1150 | 2300 | 8/15 | 7/15 | 6 – 11, 7 – 14 |

| | | | | |
|---|---|---|---|---|
| Stamm: | Swiss-Maus | | Therapiebeginn: | 7. 3. 1979 |
| Tumor: | Ehrlich-Carcinomdiploid | | Behandlungstage: | 1 × pro Woche |
| | | | Therapiedauer: | 2 Wochen |
| Transpl.-Modus: | intracerebral | | Versuchsende: | 13. 3. 1979 |
| Transpl.-Datum: | 6. 3. 1979 | | Tierzahl/Serie: | 15 |

Beispiel 6

Vergleich der Kombinationen zwischen VM 26 mit Sarkosin resp. Sarkosinanhydrid (Sarkosin AH) beim intramuskulär implantierten, diploiden Ehrlich-Ascitestumor (Tabelle 9).

Bei dieser vergleichenden Untersuchung, in welcher Sarkosin und Sarkosinanhydrid in äquimolarer Dosierung verwendet worden sind, hat sich ergeben, dass im Gegensatz zu den Befunden beim intracerebral implantierten Ehrlich-Ascitestumor der optimale Effekt bei einem Zeitintervall der Kombinationspartner (VM 26 mit Sarkosin oder Sarkosinanhydrid) von 6 Stunden auftritt. Sarkosin und Sarkosinanhydrid bewirken bei einmaliger, wöchentlicher Gabe, in gleicher Dosierung wie in der Kombination verwendet, keine signifikante Tumorhemmung. VM 26 in Monothe-rapie verursacht in dem verwendeten Dosisbereich eine zunehmende Tumorhemmung bei ansteigender Dosis (Serie 2 bis 4). Die Kombination von VM 26 und Sarkosin erweist sich sowohl hinsichtlich der Tumorhemmung als auch der Heilungsrate der VM 26-Monotherapie als deutlich überlegen (Serie 5 bis 7), wobei auch bei der Kombination mit zunehmender VM 26-Dosis ein Ansteigen der Heilungsrate zu erkennen ist. Ähnliche Ergebnisse sind mit der Kombination von VM 26 und Sarkosinanhydrid erreichbar, wobei allerdings im unteren und mittleren Dosierungsbereich die Heilungsrate stärker zunimmt als bei der höchsten VM 26-Dosis (Serie 8 bis 10).

Die Ergebnisse sind in der folgenden Tabelle 9 zusammengefasst.

Tabelle 9

| Serie Nr. | Präparat | Einzel-dosis mg/kg | Gesamt-dosis mg/kg | Geheilt | Gestorben | Ø ± Tumor-gewicht | Ø Körpergewichts-differenz |
|---|---|---|---|---|---|---|---|
| 1 | Kontrolle | – | – | – | – | 13,9 ± 3,2 | – 12,4 |
| 2 | VM 26 i.v. | 5 | 15 | 0/15 | 0/15 | 5,8 ± 1,9 | |
| 3 | VM 26 i.v. | 10 | 30 | 0/15 | 0/15 | 3,1 ± 1,6 | |
| 4 | VM 26 i.v. | 15 | 45 | 1/15 | 0/15 | 2,6 ± 1,5 | |

Tabelle 9 (Fortsetzung)

| Serie Nr. | Präparat | Einzel-dosis mg/kg | Gesamt-dosis mg/kg | Geheilt | Gestorben | $\varnothing \pm$ Tumor-gewicht | $\varnothing$ Körpergewichts-differenz |
|---|---|---|---|---|---|---|---|
| 5 | VM 26 i.v. nach 6 h | 5 | 15 | 0/15 | 0/15 | $1{,}8 \pm 0{,}9$ | |
| | Sarkosin sc. | 178 | 534 | | | | |
| 6 | VM 26 i.v. nach 6 h | 10 | 30 | 5/15 | 0/15 | $0{,}9 \pm 0{,}5$ | |
| | Sarkosin sc. | 178 | 534 | | | | |
| 7 | VM 26 i.v. nach 6 h | 15 | 45 | 13/15 | 0/15 | $0{,}3 \pm 0{,}1$ | |
| | Sarkosin sc. | 178 | 534 | | | | |
| 8 | VM 26 i.v. nach 6 h | 5 | 15 | 3/15 | 0/15 | $1{,}0 \pm 0{,}4$ | |
| | Sarkosin AH sc. | 284 | 852 | | | | |
| 9 | VM 26 i.v. nach 6 h | 10 | 30 | 8/15 | 0/15 | $0{,}5 \pm 0{,}3$ | |
| | Sarkosin AH sc. | 284 | 852 | | | | |
| 10 | VM 26 i.v. nach 6 h | 15 | 45 | 10/15 | 0/15 | $0{,}6 \pm 0{,}3$ | |
| | Sarkosin AH sc. | 284 | 852 | | | | |

| | | | | |
|---|---|---|---|---|
| Stamm: | Swiss-Maus | | Transpl.-Datum: | 9. 3. 1979 |
| Tumor: | Ehrlich-Carci-nomdiploid | | Therapiebeginn: | 12. 3. 1979 |
| | | | Behandlungstage: | 1 × pro Woche |
| Transpl.-Modus: | intramuskulär | | Therapiedauer: | 3 Wochen |
| Tumorgewicht bei | | | Tierzahl/Serie: | 15 Mäuse |
| Behandlungsbeginn: | $\varnothing$ 1,5 g | | | |

Beispiel 7

Vergleich der Wirkung von Cisplatin mit Sarkosin einzeln und in Kombination (Ergebnisse in Tabelle 10).

Cisplatin (cis-Dichlordiaminplatin, abgekürzt CPDD) besitzt wegen der Erreichung von Langzeitremissionen (z.T. bis zu fünf Jahren nach Therapieende) bei Hodentumoren, Knochensarkomen und Prostatacarcinomen eine besondere Bedeutung.

Beim diploiden Ehrlich-Carcinom, welches weiblichen Swiss-Mäusen intramuskulär implantiert wurde, erfolgte vier Tage nach der Tumortransplantation bei einem durchschnittlichen Tumorgewicht von 2,5 g die Behandlung mit Cisplatin in verschiedener Dosierung sowie Cisplatin-Sarkosin-Kombinationen. Als Positivkontrolle verwendeten wir 120 mg/kg CPA subkutan, welches ebenso wie die verschiedenen intravenös applizierten Cisplatin-Dosierungen sowie deren Kombinationen mit Sarkosin in 14-tägigem Abstand gegeben wurde.

Aus den Ergebnissen in der Tabelle geht hervor, dass CPA (Serie 2) eine deutliche Tumorhemmung bewirkt. Cisplatin (Serie 3 bis 5) erreicht bei einer Dosierung von 8 mg/kg eine signifikante Tumorhemmung und einen völligen Tumorrückgang. Die simultane Kombination von Cisplatin und Sarkosin (Serie 6 bis 8) erweist sich der Monotherapie mit Cisplatin als überlegen. In Abhängigkeit von der verwendeten Cisplatin-Dosis zeigt sich ein deutlicher Anstieg der Tumorhemmung und der Heilungsrate. Sogar die Dosierung von 4 mg/kg Cisplatin erreicht bei Kombination mit Sarkosin (Serie 6) eine Hemmung des Tumorwachstums von mehr als 50%, um schliesslich bei Verwendung von 8 mg/kg Cisplatin mit Sarkosin (Serie 8) bei 10 von 15 Tieren zu einer Heilung und bei den nicht geheilten Tieren eine intensivere Tumorhemmung zu bewirken als Cisplatin in gleicher Dosierung bei alleiniger Anwendung (Serie 5). Sarkosin besitzt bei alleiniger Anwendung in 14-tägigem Abstand keine chemotherapeutische Wirkung (Serie 9).

Tabelle 10

| Serie Nr. | Präparat | Einzel-dosis mg/kg | Gesamt-dosis mg/kg | Geheilt | Gestorben | $\varnothing$ Tumor-gewicht | $\varnothing$ Körpergewichts-differenz |
|---|---|---|---|---|---|---|---|
| 1 | Kontrolle | – | – | – | – | $14{,}3 \pm 3{,}6$ | $-23{,}2\%$ |
| 2 | CPA sc. | 120 | 360 | – | – | $4{,}7 \pm 2{,}3$ | $-4{,}4\%$ |
| 3 | CPDD i.v. | 4 | 12 | – | – | $11{,}1 \pm 4{,}1$ | $-11{,}6\%$ |

Tabelle 10 (Fortsetzung)

| Serie Nr. | Präparat | Einzel-dosis mg/kg | Gesamt-dosis mg/kg | Geheilt | Gestorben | $\varnothing$ Tumor-gewicht | $\varnothing$ Körpergewichts-differenz |
|---|---|---|---|---|---|---|---|
| 4 | CPDD i.v. | 6 | 18 | – | – | $7,8 \pm 3,4$ | – 9,1% |
| 5 | CPDD i.v. | 8 | 24 | 1/15 | – | $4,3 \pm 3,5$ | – 8,8% |
| 6 | CPDD | 4 | 12 | | | | |
| | + i.v. | | | – | – | $6,8 \pm 4,5$ | – 3,8% |
| | Sarkosin | 178 | 534 | | | | |
| 7 | CPDD | 6 | 18 | | | | |
| | + i.v. | | | 2/15 | – | $4,5 \pm 3,2$ | – 3,2% |
| | Sarkosin | 178 | 534 | | | | |
| 8 | CPDD | 8 | 24 | | | | |
| | + i.v. | | | 10/15 | – | $2,4 \pm 2,1$ | – 4,2% |
| | Sarkosin | 178 | 534 | | | | |
| 9 | Sarkosin | 178 | 534 | – | – | $11,2 \pm 3,3$ | – 12,1% |

| | | | | |
|---|---|---|---|---|
| Stamm: | Swiss | | Therapiebeginn: | 16. 1. 1979 |
| Tumor: | Ehrlich-Carcinom | | Behandlungstage: | 1., 3., 5. Woche |
| Transpl.-Modus | im. | | Therapiedauer: | 6 Wochen |
| Tumorgewicht bei | | | Versuchsende: | 19. 2. 1979 |
| Behandlungsbeginn: | 2,5 g | | Tierzahl/Serie: | 15 Mäuse |
| Transpl.-Datum: | 12. 1. 1979 | | | |

Herstellungsbeispiel 1

Zu einer Lösung von 12 g CPA in 500 ml Ethanol wird eine gesättigte wässrige Sarkosinlösung, enthaltend 28 g Sarkosin, bei Zimmertemperatur unter kräftigem Rühren zugefügt.

Die Reaktionsmasse wird kräftig weitergerührt und dann nach 10 bis 15 Minuten gefriergetrocknet.

Der Rückstand wird dann in bekannter Weise zu Tabletten gepresst, dragiert, eingekapselt oder zu einer Injektionslösung konfektioniert, wobei eine Einzeldosis die für CPA übliche Menge von 50 mg an CPA-Anteil, kombiniert mit 117 mg Sarkosin, enthält.

Herstellungsbeispiel 2

In gleicher Weise wie beim Herstellungsbeispiel 1 wurden 18 g Sarkosin, als konzentrierte wässrige Lösung, mit 8 g Adriamycin, gelöst in 500 ml Ethanol, umgesetzt. Durch Gefriertrocknen wird das Additionsprodukt isoliert. Eine Einheitsdosis erhält den Adriamycin/Sarkosinkomplex in einem Verhältnis von 50 mg Adriamycin zu 1125 mg Sarkosin.

Herstellungsbeispiel 3

29 g Sarkosinanhydrid in 250 ml Wasser wurden in eine 2%ige ethanolische Lösung von 6 g VM 26 eingebracht. Nach mehrminütigem kräftigem Rühren und Stehenlassen wurde die erhaltene Lösung gefriergetrocknet und in bekannter Weise zu einem Injektionspräparat konfektioniert, das pro Einheitsdosis 30 mg an VM-Anteil und 1450 mg an Sarkosinanhydridanteil enthielt.

Herstellungsbeispiel 4

8 g Cisplatin wurden als 1,5%ige wässrige Lösung, die gleichzeitig 0,9%ig an NaCl war, unter kräftigem Rühren in eine wässrige Lösung von 178 g Sarkosin in 500 ml Wasser eingebracht. Nach 5 bis 10 minütigem weiterem kräftigem Rühren wurde das Reaktionsgemisch gefriergetrocknet und in bekannter Weise zu einem Injektionspräparat konfektioniert, das pro Einheitsdosis 80 mg CPDD enthielt.

Patentansprüche
für die Vertragsstaaten BE CH FR GB IT LU NL SE

1. N-Niedrigalkylglycine mit 1 bis 4 Kohlenstoffatomen im N-Alkylteil in Form der Säureamide sowie Sarkosinanhydrid als alleiniger Wirkstoff, im Gemisch miteinander oder in Kombination mit bekannten tumorhemmenden Verbindungen zur Anwendung bei der Heilung von Tumoren.

2. Sarkosinanhydrid, N-Methylglycinamid und N-n-Propylglycinamid zur Anwendung bei der Heilung von Tumoren nach Anspruch 1.

3. Sarkosin in Kombination mit bekannten tumorhemmenden Verbindungen zur Anwendung bei der Heilung von Tumoren.

4. Antineoplastische Alkylantien, intercalierende Stoffe und/oder Antimetabolite als bekannte tumorhemmende Verbindungen zur kombinierten Anwendung bei der Heilung von Tumoren nach Ansprüchen 1 und 3.

5. Mittel mit tumorhemmender und/oder die Aktivität bekannter tumorhemmender Verbindungen synergistisch unterstützender Wirkung zur Verwendung nach Ansprüchen 1 bis 4, gekennzeichnet durch einen Gehalt an N-Niedrigalkylglycinen mit 1 bis 4 Kohlenstoffatomen im N-Alkylteil in Form der Säureamide sowie von Sarkosinanhydrid einzeln, im Gemisch bzw. zusammen mit bekannten tumorhemmenden Verbindungen als aktive Komponente, ggf. mit üblichen Träger- und Hilfsstoffen.

6. Mittel mit tumorhemmender und/oder die Aktivität bekannter tumorhemmender Verbindungen synergistisch unterstützender Wirkung zur kombinierten Verwendung nach Ansprüchen 1 bis 4, gekennzeichnet durch einen Gehalt an Sarkosin im Gemisch mit bekannten tumorhemmenden Verbindungen als aktive Komponente, ggf. mit üblichen Träger- und Hilfsstoffen.

7. Verfahren zur Herstellung von Mitteln nach Ansprüchen 5 und 6 zur Anwendung bei der Heilung von Tumoren, dadurch gekennzeichnet, dass man a) wenigstens ein Mitglied der Gruppe N-Niedrigalkylglycine mit 1 bis 4 Kohlenstoffatomen im N-Alkylteil in Form der Säureamide, Sarkosin und Sarkosinanhydrid mit b) einem bekannten Antitumormittel kombiniert, indem man eine wirksame Menge von jeweils a) und b) in innigen Kontakt miteinander bringt und das erhaltene Produkt isoliert.

8. Verfahren nach Anspruch 7, dadurch gekennzeichnet, dass 2:1 bis 50:1, insbesondere 2:1 bis 10:1 Gewichtsteile an a) und b) kombiniert werden.

9. Verfahren nach Anspruch 7 oder 8, dadurch gekennzeichnet, dass $\frac{1}{10}$ bis $-\frac{1}{20}$ der $LD_{50}$ von b) mit $-\frac{1}{20}$ bis $-\frac{1}{100}$ der $LD_{50}$ von a) zur Herstellung einer Einheitsdosis kombiniert werden.

10. Verfahren nach Anspruch 7 bis 9, dadurch gekennzeichnet, dass das Produkt eingekapselt oder granuliert wird, gegebenenfalls unter weiterer Zugabe von Wirksubstanz a) oder b) in einer Form mit verzögerter Abgabe an den Körper.

**Patentansprüche**
**für den Vertragsstaat AT**

1. Verfahren zur Herstellung eines Mittels mit tumorhemmender und/oder die Aktivität bekannter tumorhemmender Verbindungen synergistisch unterstützender Wirkung, dadurch gekennzeichnet, dass man a) wenigstens ein Mitglied der Gruppe N-Niedrig-alkylglycine mit 1 bis 4 Kohlenstoffatomen im N-Alkylteil in Form der Säureamide, Sarkosin und Sarkosinanhydrid mit b) einem üblichen Antitumormittel kombiniert, indem man eine wirksame Menge von jeweils a) und b) in innigen Kontakt miteinander bringt und das erhaltene Produkt isoliert.

2. Verfahren nach Anspruch 1, dadurch gekennzeichnet, dass der innige Kontakt bewirkt wird, indem zumindest eine der Komponenten a) und b) als Lösung eingesetzt wird, dem die andere Komponente in Pulverform oder in Form einer damit verträglichen Lösung, vorzugsweise im gleichen Lösungsmittel, zugeben wird.

3. Verfahren nach Anspruch 1 oder 2, dadurch gekennzeichnet, dass 2:1 bis 50:1, insbesondere 2:1 bis 10:1 Gewichtsteile an a) und b) kombiniert werden.

4. Verfahren nach Anspruch 1 oder 2, dadurch gekennzeichnet, dass $-\frac{1}{10}$ bis $-\frac{1}{20}$ der $LD_{50}$ von b) mit $-\frac{1}{20}$ bis $-\frac{1}{100}$ der $LD_{50}$ von a) zur Herstellung einer Einheitsdosis kombiniert werden.

5. Verfahren nach Anspruch 1 bis 4, dadurch gekennzeichnet, dass die Isolierung des Produktes in an sich bekannter Weise, insbesondere durch Gefriertrocknung oder Vakuumtrocknung erfolgt.

6. Verfahren nach Anspruch 1 bis 4, dadurch gekennzeichnet, dass das Produkt eingekapselt oder granuliert wird, gegebenenfalls unter weiterer Zugabe von Wirksubstanz a) oder b) in einer Form mit verzögerter Abgabe an den Körper.

7. Verfahren nach Anspruch 1 bis 4, dadurch gekennzeichnet, dass das Produkt zu einem Injektionspräparat, insbesondere einer Injektionslösung konfektioniert wird.

**Claims for the Contracting states BE, CH, FR, GB, IT, LU, NL, SE**

1. N-lower alkyl glycines with 1 to 4 carbon atoms in the N-alkyl part in the form of acid amides as well as sarcosine anhydride as sole active substance, in mixtures with each other or in combination with known tumor inhibiting compounds for use in the treatment of tumors.

2. Sarcosine anhydride, N-methylglycineamide and N-n-propylglycine amide for use in the treatment of tumors in accordance with claim 1.

3. Sarcosine in combination with known tumor inhibiting compounds for use in the treatment of tumors.

4. Antineoplastic alkylantiene, intercalating substances and/or antimetabolites as known tumor inhibiting compounds in combinations used in the treatment of tumors in accordance with claims 1 and 3.

5. Agents with tumor inhibiting activity and/or which synergistically support tumor inhibiting compounds for use according to claims 1 to 4, characterised by a content of N-lower alkyl glycines with 1 to 4 carbon atoms in the N-alkyl part in the form of acid amide as well as sarcosine anhydride alone, as mixtures or together with known tumor inhibiting compounds as active components with usual carrier- and auxiliary-substances if desired.

6. Agents with tumor inhibiting activity and/or which synergistically support tumor inhibiting compounds for combined use according to claims 1 to 4, characterised by a content of sarcosine mixed with known tumor inhibiting compounds as active components with usual carrier- and auxiliary-substances if desired.

7. Process for the preparation of agents according to claims 5 and 6 for use in the treatment of tumors, characterised in that a) at least one member of the group N-lower alkyl glycine having 1 to 4 carbon atoms in the N-alkyl part in the form of acid amide, sarcosine and sarcosine anhydride is combined with b) a known anti-tumor agent, by bringing an effective amount of each of a) and b) into intimate contact with each other and isolating the product obtained thereby.

8. Process according to claim 7, characterised in that a) and b) are combined by 2:1 to 50:1, preferably 2:1 to 10:1 parts by weight.

9. Process according to claim 7 or 8, characterised in that $1/10$ to $1/20$ of the $LD_{50}$ of b) are combined with $1/20$ to $1/100$ of the $LD_{50}$ of a) in a standard dose.

10. Process according to claim 7 to 9, characterised in that the product is encapsuled or granulated, if desired with further addition of active substance a) or b) in a form with delayed release in the body.

**Claims for the Contracting state: AT**

1. Process for the preparation of an agent with tumor inhibiting activity and/or which synergistically support tumor inhibiting compounds characterised in that a) at least one member of the group N-lower alkyl glycine having 1 to 4 carbon atoms in the N-alkyl part in the form of acid amide, sarcosine and sarcosine anhydride is combined with b) an ordinary anti-tumor agent by bringing an effective amount of each of a) and b) into intimate contact with each other and isolating the product obtained thereby.

2. Process according to claim 1, characterised in that the intimate contact is effected by using at least one of the components a) and b) as a solution, adding the other component in powder form or in the form of compatible solution, preferably in the same solvent.

3. Process according to claim 1 or 2, characterised in that a) and b) are combined by 2:1 to 50:1, preferably 2:1 to 10:1 parts by weight.

4. Process according to claim 1 or 2, characterised in that $1/10$ to $1/20$ of the $LD_{50}$ of b) are combined with $1/20$ to $1/100$ of the $LD_{50}$ of a) in the preparation of a standard dose.

5. Process according to claim 1 to 4, characterised in that the isolation of the product is carried out in known manner, particularly by freeze drying or vacuum drying.

6. Process according to claim 1 to 4, characterised in that the product is encapsulated or granulated, if desired with further addition of active substance a) or b) in a form with delayed release in the body.

7. Process according to claim 1 to 4, characterised in that the product is manufactured from an injection preparation, particularly an injection solution.

**Revendications pour les Etats contractants: BE, CH, FR, GB, IT, LU, NL, SE**

1. Glycinamides substituées sur l'azote par un groupe alkyle inférieur, contenant 1 à 4 atomes de carbone dans le fragment N-alkyle, sous la forme des amides d'acide, et aussi anhydride de sarcosine en tant que substance active unique, en mélange entre eux ou en association avec des composés connus inhibiteurs de tumeurs, pour l'utilisation dans la guérison de tumeurs.

2. Anhydride de sarcosine, N-méthyl-glycinamide et N-(n-propyl)-glycinamide pour l'utilisation dans la guérison de tumeurs selon la revendication 1.

3. Sarcosine en association avec des composés inhibiteurs de tumeurs, pour l'utilisation dans la guérison de tumeurs.

4. Agents d'alkylation anti-néoplastiques, substances d'intercalation et/ou antimétabolites, en tant que composés connus inhibiteurs de tumeurs, pour l'utilisation conjointe dans la guérison de tumeurs selon les revendications 1 et 3.

5. Agents ayant une action inhibitrice des tumeurs et/ou favorisant par synergie l'activité de composés connus inhibiteurs de tumeurs, pour utilisation selon les revendications 1 à 4, caractérisés par une teneur en glycines substituées sur l'azote par un groupe alkyle inférieur, contenant 1 à 4 atomes de carbone dans le fragment alkyle, sous la forme des amides d'acide, ainsi qu'en anhydride de sarcosine, isolément, en mélange ou conjointement avec des composés connus inhibiteurs de tumeurs, comme constituant actif, éventuellement avec des véhicules et adjuvants usuels.

6. Agent ayant une action inhibitrice des tumeurs et/ou une action favorisant par synergie l'activité de composés connus inhibiteurs de tumeurs, pour l'utilisation en association selon les revendications 1 à 4, caractérisé par une teneur en sarcosine, en mélange avec des composés connus inhibiteurs de tumeurs, comme constituant actif, éventuellement avec des véhicules et adjuvants usuels.

7. Procédé de préparation d'agents selon les revendications 5 et 6, pour l'utilisation dans la guérison de tumeurs, caractérisé par le fait que l'on associe a) au moins un membre du groupe des glycines substituées sur l'azote par un groupe alkyle inférieur, contenant 1 à 4 atomes de carbone dans le fragment alkyle, sous la forme des amides d'acide, de la sarcosine et de l'anhydride de sarcosine, et b) un agent antitumoral connu, en mettant en contact intime entre eux une quantité efficace de chacun de a) et b), et que l'on isole le produit obtenu.

8. Procédé selon la revendication 7, caractérisé par le fait que l'on associe entre 2:1 et 50:1 parties en poids, en particulier entre 2:1 et 10:1 parties en poids de a) et b).

9. Procédé selon l'une des revendications 7 et 8, caractérisé par le fait que l'on associe $1/10$ à $1/20$ de la $DL_{50}$ de b) et $1/20$ à $1/100$ de la $DL_{50}$ de a) pour préparer une dose unité.

10. Procédé selon les revendications 7 à 9, caractérisé par le fait que l'on encapsule ou que l'on granule le produit, éventuellement en ajoutant un supplément de substance active a) ou b), sous une forme à libération retardée dans l'organisme.

**Revendications pour l'Etat contractant: AT**

1. Procédé de préparation d'un agent ayant une action inhibitrice des tumeurs et/ou une action favorisant par synergie l'activité de composés connus inhibiteurs de tumeurs, caractérisé par le fait que l'on associe a) au moins un membre du groupe des glycines substituées sur l'azote par un groupe alkyle inférieur, contenant 1 à 4 atomes de carbone dans le fragment alkyle, sous la forme des amides d'acide, de la sarcosine et de l'anhydride de sarcosine, et b) un agent antitumoral connu, en mettant en contact intime entre eux une quantité efficace de chacun de d) et b), et que l'on isole le produit obtenu.

2. Procédé selon la revendication 1, caractérisé par le fait que l'on provoque le contact intime en introduisant au moins un des constituants a) et b) sous forme solution à laquelle on ajoute l'autre constituant sous forme de poudre ou sous la forme d'une solution compatible avec la première, de préférence dans le même solvant.

3. Procédé selon l'une des revendications 1 et 2, caractérisé par le fait que l'on associe entre 2:1 et 50:1, en particulier entre 2:1 et 10:1 parties en poids de a) et b).

4. Procédé selon l'une des revendications 1 et 2, caractérisé par le fait que l'on associe $1/10$ à $1/20$ de la $DL_{50}$ de b) et $1/20$ à $1/100$ de la $DL_{50}$ de a) pour préparer une dose unité.

5. Procédé selon les revendications 1 à 4, caractérisé par le fait que l'isolement du produit s'effectue de manière en elle-même connue, en particulier par lyophilisation ou séchage sous vide.

6. Procédé selon les revendications 1 à 4, caractérisé par le fait que l'on encapsule ou que l'on granule le produit, éventuellement en ajoutant un supplément de substance active a) ou b), sous une forme à libération retardée dans l'organisme.

7. Procédé selon les revendications 1 à 4, caractérisé par le fait que l'on conditionne le produit en une composition injectable, en particulier en une solution injectable.